# EUROPEAN PATENT APPLICATION

(11) **EP 2 666 842 A1**
(43) Date of publication of application: **27.11.2013**
(21) Application number: 12168845.1
(22) Date of filing: 22.05.2012
(51) Int. Cl.: C09K 17/00, C12N 1/00

(54) **A method for producing a scour protection or support for a submarine structure**

(71) Applicant: Siemens Aktiengesellschaft, 80333 München (DE)
(72) Inventor: Oestergaard, Thomas, 6000 Kolding (DK)

(57) **Abstract**

According to the method, bacteria with the ability to precipitate calcite are added to a portion of submarine floor for solidification of the same to form the scour protection or support.

In this manner, a scour protection or support for a submarine structure can be created without the need for moving large amounts of gravel or rocks.

Further, an apparatus for producing a scour protection or support for a submarine structure is provided.

## Description

The present invention relates to a method for producing a scour protection or support for a submarine structure and an apparatus.

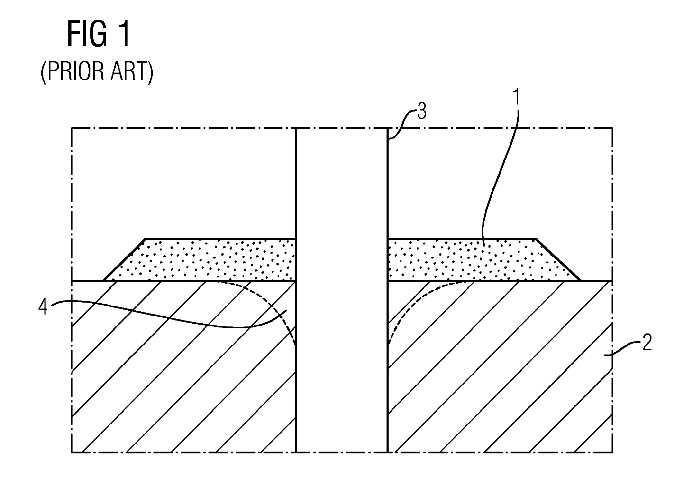

Fig. 1 shows a scour protection 1 according to the prior art. The scour protection, also known as erosion protection, is made of layers of rocks and gravel dumped on a portion 2 of submarine floor in a circular area around a submarine structure 3. The submarine structure 3 may be a pile extending through the scour protection 1 into the portion 2 of submarine floor at its one end. At its other end (not shown), the pile 3 may be connected to a platform above the surface of the sea or at sea level. The scour protection 1 prevents the sea from eroding the portion 2 in an area 4 indicated by dotted lines in the vicinity of the pile 3. Such erosion may destabilize the pile 3 in the long term. Instead of the scour protection 1, a pile 3 of larger dimensions may be used, which however also adds cost.

It is one object of the present invention to provide an improved method for producing a scour protection or support for a submarine structure. In particular, it is desired to provide an efficient process of making the scour protection or support.

Accordingly, a method for producing a scour protection or support for a submarine structure is provided, wherein bacteria with the ability to precipitate calcite are added to a portion of submarine floor for solidification of the same to form the scour protection or support.

When the bacteria are added to the portion of submarine floor, the bacteria start precipitating calcite. The bacteria convert calcium into calcite in accordance with the following equation:

Ca²⁺ + 2HCO₃⁻ => CaCo₃ + H₂O + CO₂

The calcite and at least some of the particles of the submarine floor making up the portion, for example sand, gravel or other granular material, combine to form a rigid structure. In other words, the stiffness of the submarine floor is increased. The formed structure is resistant to scouring. In addition or alternatively, the structure provides mechanical support to the submarine structure, i.e. transmits forces from the submarine structure into the submarine floor to hold the submarine structure in place. For example, deformation of the submarine floor induced from the submarine structure, in particular due to tilting of the submarine structure, may be prevented.

Generally, the calcite will take some time to grow. For example, it may take up to 5 days until the structure has reached its full strength. A lower strength may be obtained within shorter time.

The structure (i.e. the scour protection or support) formed through solidification of the portion of submarine floor has a compressive strength and/or modulus of elasticity that is at least higher than the compressive strength and/or modulus of elasticity of the submarine floor surrounding the structure. The compressive strength of the fully hardened structure may lie between 10 and 30 MPa. The stiffness of the fully hardened material may be up to that of marble.

By way of the method, it may not be necessary any more to move large amounts of earth, i.e. gravel and rocks, to create a scour protection or support. Rather, the submarine floor that is already present is reinforced by calcite produced by the bacteria.

Hence, a scour protection, a support or both may be produced easily. The scour protection or the support can be one and the same structure.

"Submarine" encompasses sea water and fresh water applications of the method according to the present invention. "Submarine floor" thus encompasses a seabed.

According to an embodiment, a mixture of the bacteria and urea and/or calcium is added to the portion of submarine floor. "Mixture" may refer to a solution of the bacteria. In some instances, calcium or other minerals relevant to the chemical process may already be present in the portion of submarine floor. Then, it may be sufficient to add a mixture of bacteria and urea only to the portion of submarine floor. The bacteria will turn the calcium present in the portion of submarine floor into calcite. Generally speaking, the mixture may be adjusted to the concentration of minerals present in the submarine floor to avoid having minerals in free form.

Preferably, the mixture is balanced so that all urea and calcium present (either in the mixture or in the portion of submarine floor) has been turned into calcite, when the bacteria die.

According to a further embodiment, the bacteria are of the genus sporosarcina. In particular, the bacteria are sporosarcina pasteurii. Such bacteria are well suited to convert calcium to calcite.

According to a further embodiment, the portion of submarine floor comprises a granular material. The granular material may be sand, gravel or other fine particles with a diameter smaller than 1 mm, preferably.

According to a further embodiment, the bacteria or mixture is injected into the portion of submarine floor. The bacteria or mixture is easily added to the portion of submarine floor by injection. Yet, other ways of adding the bacteria or mixture to the submarine floor are feasible.

According to a further embodiment, at least one hollow needle is used for injecting the bacteria or mixture. The needle may only have an opening at its tip. In addition or alternatively, the needle may have openings along its length.

According to a further embodiment, the at least one needle is moved horizontally in between injections to cover the portion of submarine floor. Thus, the needle is lowered into the portion of submarine floor in a first step. Then, the bacteria or mixture is injected into the portion. Hereafter, the needle is raised again. In a further step, the needle is moved horizontally. This process is repeated to cover a large portion of submarine floor, for example 2 to 100 m².

According to a further embodiment, the submarine structure is placed underneath, placed into, placed on top or connected to the portion of submarine floor after or before adding the bacteria or mixture or after or before solidification of the portion of submarine floor.

According to a further embodiment, the submarine structure comprises a pile, a gravity-based structure, a pipe or a cable. The submarine structure is at least partially under water when connected to or in contact with the scour protection or support. A "gravity-based structure" is a support structure held in place by gravity. A common application for a gravity-based structure is an offshore wind turbine. The pipe may be an oil pipe. The cable may be a power cable or a data cable. The submarine structure may also comprise anchors which are drilled or otherwise anchored in the scour protection or support.

According to a further embodiment, first, the portion of submarine floor is solidified to form the scour protection or support and, second, the pile is driven into the thus formed scour protection or support. The pile may extend vertically to the scour protection or support after being driven into the same.

According to a further embodiment, the scour protection or support is formed underneath a top layer of submarine floor. This will allow the bacteria to be protected from the sea, for example currents, while producing calcite. This may increase the bacteria's efficiency. Also, temperatures below the top surface of submarine floor may be higher, which may again improve the productivity of the bacteria.

According to a further embodiment, the top layer is removed and the submarine structure is placed into, placed on top of and/or connected to the exposed scour protection or support. In this manner, a hole in the submarine floor with a rigid bottom may be formed. This hole is well-suited to receive a submarine structure, in particular a gravity-based structure.

According to a further embodiment, an excavation is made in a further portion of submarine floor, the submarine structure is placed inside the excavation, the excavation is covered with the portion of submarine floor and the bacteria or mixture is added to the portion for solidification. In this manner, structures may be easily buried inside the submarine floor. This process is well suited for cables or pipes, in particular. The thus formed scour protection protects the submarine structure inside the submarine floor from the top (and possibly from the side).

According to a further embodiment, the excavation is formed by jetting or ploughing and/or the submarine structure is formed as a cable or pipe. In this manner, the excavation can be made easily.

Further, an apparatus for producing a scour protection or support for a submarine structure in accordance with the method of the present invention is provided. The apparatus comprises a device configured for adding bacteria with the ability to precipitate calcite to a portion of submarine floor for solidification of the same to form the scour protection or support.

The device may be formed as a needle, for example.

Further objects, features and advantages of the present invention become apparent from the subsequent description and dependent claims, taken in conjunction with the accompanying drawings, in which:
Fig. 1 shows a prior art scour protection;
Fig. 2 is a schematic diagram of a needle according to an embodiment;
Fig. 3 is a schematic diagram of a needle according to a further embodiment;
Fig. 4 is a schematic diagram of an embodiment of an apparatus;
Fig. 5 shows the apparatus of Fig. 4 being employed to inject a portion of submarine floor at a first point in time according to an embodiment;
Fig. 6 shows the apparatus of Fig. 5 at a second point in time;
Fig. 7 is a schematic diagram of a pile connected to a scour protection and support formed in the steps according to Figs. 5 and 6 according to an embodiment;
Fig. 8 is a top view of multiple portions of submarine floor according to an embodiment;
Fig. 9 is a schematic diagram of a support formed underneath a top layer of submarine floor according to an embodiment;
Fig. 10 is a schematic drawing of a gravity-based structure set on the support shown in Fig. 9 according to an embodiment; and
Fig. 11 shows a schematic diagram of a cable protected under a scour protection according to an embodiment.

In the Figures, like reference numerals designate like or functionally equivalent elements, unless otherwise indicated.

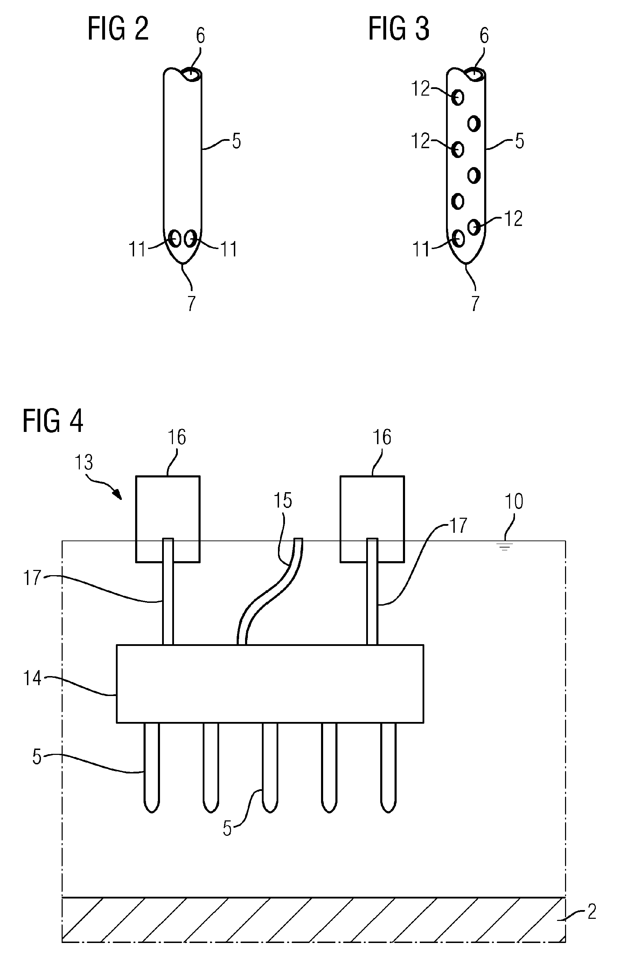

Fig. 2 is a schematic diagram of a hollow needle 5.

At its one end, the needle 5 is formed with an inlet opening 6 to introduce a mixture of bacteria, urea and calcium into the needle 5. At its other end, the needle 5 has a tip 7. The tip 7 is adapted to break into a portion 2 of submarine floor shown in Fig. 4. The needle 5 may be provided with one or more outlet openings 11 formed inside the tip 7.

Fig. 3 shows a variation of the hollow needle 5 of Fig. 2.

In addition to the outlet openings 11, the hollow needle 5 has outlet openings 12 spaced along its length.

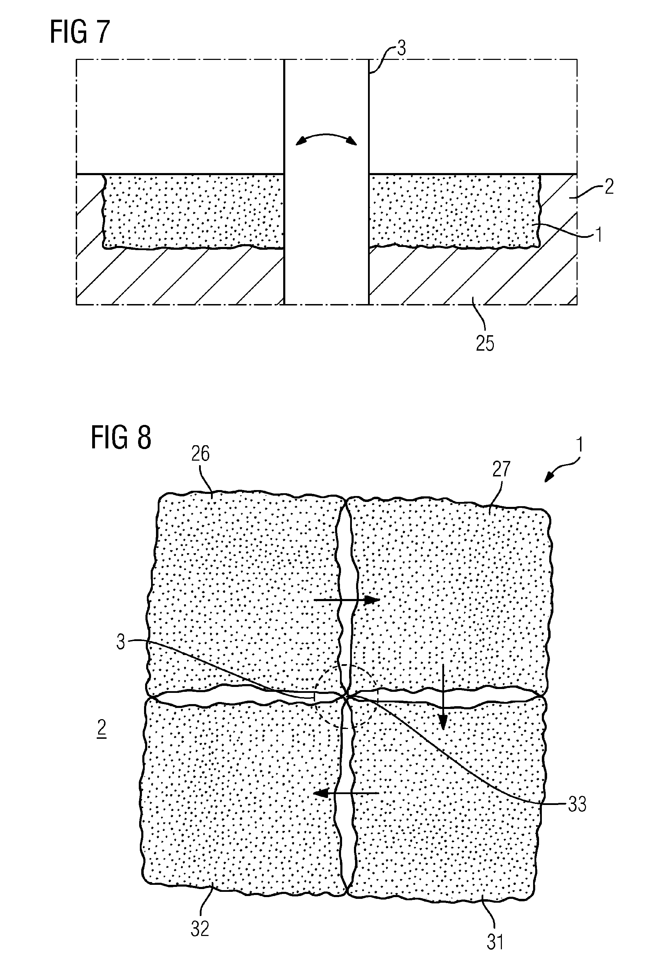

Fig. 4 shows an apparatus 13 for producing a scour protection and support 1 for a submarine structure 3 (see Fig. 7, for example).

The apparatus 13 has a stable base 14. A plurality of needles 5, which may be formed as described in connection with Fig. 2 or 3, are arranged underneath the base 14 and fastened thereto. The inlet openings 6 (not shown in Fig. 4) are connected to a supply pipe 15 supplying the mixture of bacteria, urea and calcium. The pipe 15 is in turn connected to a pump and a tank. The tank holds a large supply of the mixture. The pump and the tank are typically arranged on a ship or other floating platform. The level of sea water is indicated at 10. Further, the apparatus 13 comprises one or more actuators 16, which may be formed as hydraulic cylinder/piston arrangements, for example. Pistons 17 of the actuator 16 are connected to the base 14 in order to drive the needles 5 into and out of the portion 2 of submarine floor. The actuators 16 may be supported by a ship or other floating platform.

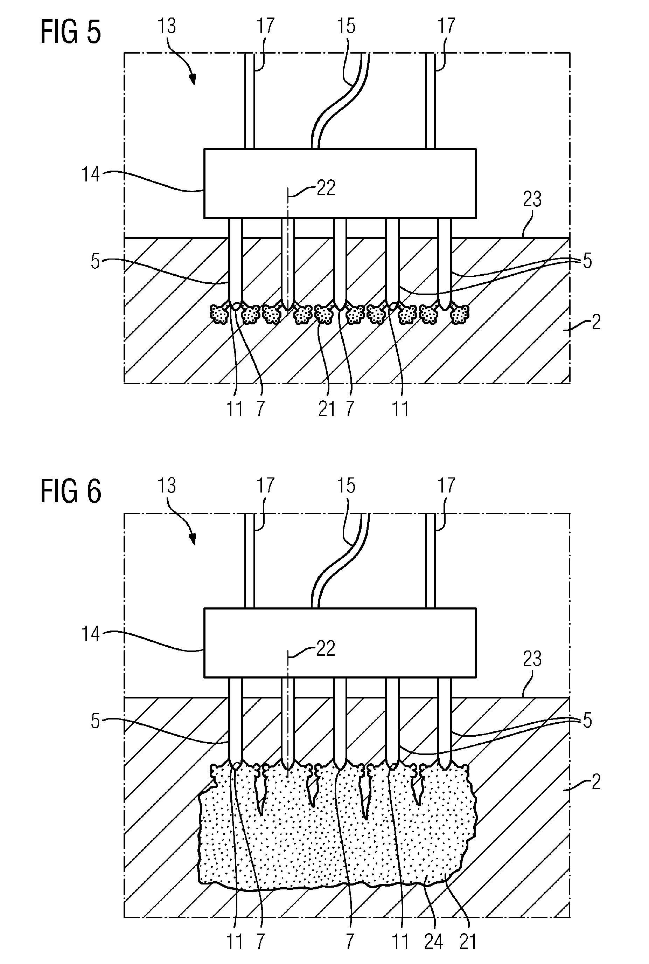

Figs. 5 and 6 illustrate the process of injecting the mixture (indicated at 21) into the portion 2 of submarine floor using the apparatus 13.

The actuators 16 push the needles 5 into the portion 2 of submarine floor. Whilst pushing, the tips 7 of the needles 5 pierce into the portion 2 of submarine floor. As the needles 5 are being pushed down, they may also be turned around a respective longitudinal axis 22. Thus, a kind of drilling motion results for each needle 5, which makes penetration of the portion 2 of submarine floor easier. Once the needles 5 have reached the desired depth inside the portion 2 of submarine floor, the mixture 21 is supplied by the pipe 15 to each needle 5. In the example according to the Figs. 5 and 6, the needles 5 are configured as explained in connection with Fig. 2. Thus, the mixture 21 exits from the outlet openings 11 into the portion 2 of submarine floor. The bacteria are of the genus sporosarcina. In particular, the bacteria are sporosarcina pasteurii. The composition of the mixture 21 is balanced such that the amount of bacteria matches the amount of urea and the amount of calcium. As a result, all the urea and calcium is converted to calcite, when the bacteria die. The mixture 21 can be provided with a high pressure to improve distribution of the mixture 21 in the portion 2. The portion 2 is made up of a granular material, for example sand 24. Thus, a suspension of sand 24 and mixture 21 is formed.

The needles 5 are slowly raised, the mixture 21 hence being distributed over the portion 2 in the upwards direction until just under the surface 23 of the portion 2 of submarine floor.

As time passes, the bacteria convert urea and calcium to calcite. As a result, a finely dispersed structure of calcite forms around sand particles 24. Thus, the calcite binds the sand together. The result is a rigid structure, which may serve as a scour protection and support 1, as shown in Fig. 7.

Fig. 7 is a schematic diagram of a scour protection and support 1 and a pile 3.

Once the portion 2 of submarine floor has fully solidified to form the scour protection and support 1, which is typically when all the bacteria have died, the pile 3, for example a monopile, is driven through the scour protection 1 into the underlying submarine floor 25. Because of its rigidness, the scour protection and support 1 prevents scouring and tilting of the pile 3 in the direction of the double arrow as indicated in Fig. 7. The pile 3 is only one example of a submarine structure that can be protected against scouring and supported by the scour protection and support 1.

In other embodiments of the method described above, the portion 2 may already comprise the calcium. In this rare case, calcium does not need to be added into the mixture 21 supplied by the pipe 15. Also, the pile 3 may already be driven into the portion 2 of submarine floor, and the mixture 21 is injected into the portion 2 afterwards.

Fig. 8 shows a top view of a portion 2 of submarine floor.

A scour protection 1 has been formed by moving the apparatus 13 of Fig. 4 over a first area 26 lowering the apparatus 13 and injecting the mixture 21 into the area 26, lifting the apparatus 13 and moving the apparatus 13 to a second area 27 horizontally, as indicated by the arrow in Fig. 8. This process is repeated for areas 31 and 32 so as to form a square or rectangular shaped scour protection and support 1 when viewed from above. The total area thus covered is a larger area than the area covered by the needles 5 of a single apparatus 13. Of course, this process can also be applied to produce scour protections and supports 1 of a different shape.

A submarine structure, for example the pile 3 of Fig. 7, may be connected to the scour protection and support 1 at the center 33 formed by the four areas 26, 27, 31 and 32 at their respective edges. The pile 3 may be arranged in the same manner as explained in connection with Fig. 7.

The large area covered by the areas 26, 27, 31 and 32 allows a slimmer design of the pile 3 since the hence formed larger scour protection and support 1 provides high stiffness.

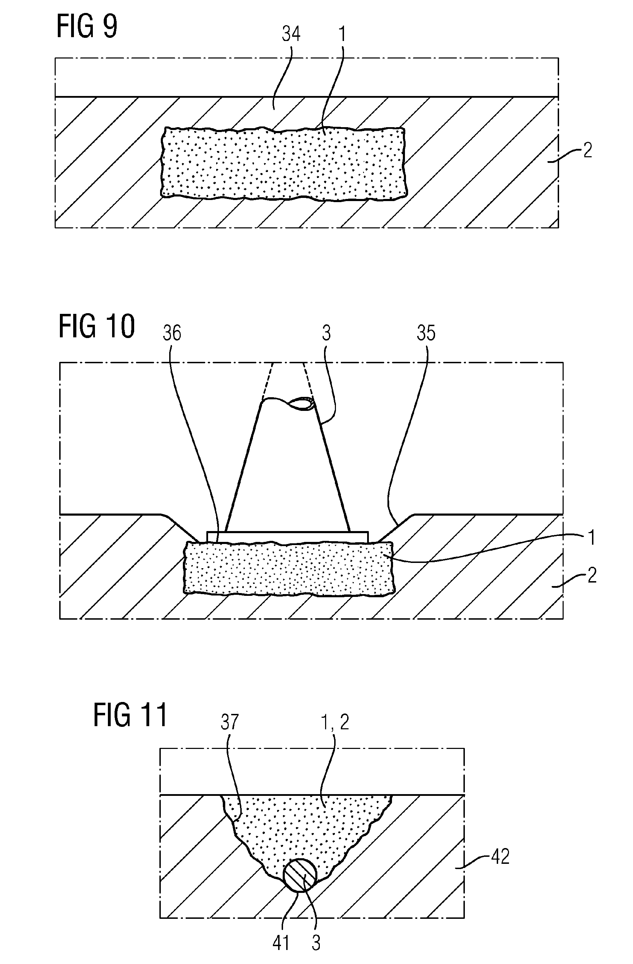

Fig. 9 and Fig. 10 illustrate in a schematic drawing respectively a process of installing a submarine structure in the form of a gravity-based structure 3.

A support 1 in Fig. 9 is created by using the process explained in connection with the Figs. 5 and 6. However, the support 1 is produced underneath a top layer 34 of submarine floor. Once the support 1 has fully solidified, the top layer 34 is partially removed to form a hole 35, which may be cone-shaped. The bottom of the hole 35 is formed by the top surface 36 of the support 1. The gravity-based structure 3 is set onto a top surface 36 and may, in addition, be bolted or otherwise anchored to the support 1. The gravity-based structure 3 may be supporting an oil platform or wind turbine (not shown).

The top layer 34 protects the process of forming the calcite from the submarine, in particular currents. Also, higher temperatures encountered deeper inside the submarine floor may be beneficial to the process of forming the calcite.

Fig. 11 is a schematic drawing of a submarine structure in the form of a cable or pipe 3 protected by a scour protection 1 from the submarine.

Initially, an excavation 37 is formed in a portion 42 of submarine floor. This can be done by jetting or ploughing. Then, the cable or pipe 3 is placed at the bottom 41 of the excavation 37. The pipe or cable 3 is thereafter covered again with a portion 2 of sea floor, which may correspond to the initially excavated material. In a further step or integrated in the step of jetting, the portion 2 is injected with the mixture 21 in a process as described for Figs. 5 and 6, or similar. After solidification of the portion 2, a scour protection 1 is formed which protects the pipe or cable 3. Instead of the pipe or cable 3, other submarine structures may also be covered by the same method.

Although the present invention has been described in accordance with preferred embodiments, it is obvious for a person skilled in the art that modifications are possible in all embodiments.

## Claims

1. A method for producing a scour protection or support (1) for a submarine structure (3), wherein bacteria with the ability to precipitate calcite are added to a portion (2) of submarine floor for solidification of the same to form the scour protection or support (1).

2. The method according to claim 1, wherein a mixture (21) of the bacteria and urea and/or calcium is added to the portion (2) of submarine floor.

3. The method according to claim 1 or claim 2, wherein the bacteria are of the genus sporosarcina.

4. The method according to one of claims 1 to 3, wherein the portion (2) of submarine floor comprises a granular material (24).

5. The method according to one of claims 1 to 4, wherein the bacteria or mixture (21) is injected into the portion (2) of submarine floor.

6. The method according to claim 5, wherein at least one hollow needle (5) is used for injecting the bacteria or mixture (21).

7. The method according to claim 6, wherein the at least one needle (5) is moved horizontally in between injections to cover the portion (26, 27, 31, 32) of submarine floor.

8. The method according to one of claims 1 to 7, wherein the submarine structure (3) is placed underneath, placed into, placed on top of and/or connected to the portion (2) of submarine floor after or before adding the bacteria or mixture (21) or after or before solidification of the portion (2) of submarine floor.

9. The method according to one of claims 1 to 8, wherein the submarine structure (3) comprises a pile, a gravity-based structure, a pipe or a cable.

10. The method according to claim 9, wherein, first, the portion (2) of submarine floor is solidified to form the scour protection or support (1), and, second, the pile (3) is driven into the formed scour protection or support (1).

11. The method according to one of claims 1 to 10, wherein the scour protection or support (1) is formed underneath a top layer (34) of submarine floor.

12. The method according to claim 11, wherein the top layer (34) is removed and the submarine structure (3) is placed into, placed on top of or connected to the exposed scour protection or support (1).

13. The method according to one of claims 1 to 9, wherein an excavation (37) is made in a further portion (42) of submarine floor, the submarine structure (3) is placed inside the excavation (37), the excavation (37) is covered with the portion (2) of submarine floor, the bacteria or mixture (21) is added to the portion (2) of submarine floor for solidification.

14. The method according to claim 13, wherein the excavation (37) is formed by jetting or ploughing and/or the submarine structure (3) is formed as a cable or pipe.

15. An apparatus (13) for producing a scour protection or support (1) for a submarine structure (3) in accordance with the method of one of claims 1 to 14, comprising a device (5) for adding bacteria with the ability to precipitate calcite to a portion (2) of submarine floor for solidification of the same to form the scour protection or support (1).
